# EUROPEAN PATENT SPECIFICATION

(11) **EP 3 244 968 B1**
(45) Date of publication and mention of the grant of the patent: **30.08.2023**
(21) Application number: 16737651.6
(22) Date of filing: 08.01.2016
(51) Int. Cl.: A61K 31/436, A61K 31/4025, A61K 31/422, A61K 31/497, A61K 31/4985, A61K 31/505, A61K 31/519, A61K 31/53, A61K 31/5575, A61K 31/506, A61K 31/5585, A61K 45/06, A61P 11/00

(54) **COMBINATION THERAPY FOR PULMONARY HYPERTENSION**
KOMBINATIONSTHERAPIE FÜR PULMONALE HYPERTONIE
TRAITEMENT COMBINÉ POUR L'HYPERTENSION PULMONAIRE

(30) Priority: 13.01.2015 US 201562103020 P
(43) Date of publication of application: 22.11.2017
(73) Proprietor: Vivus LLC, Campbell, CA 95008 (US)
(72) Inventor: GU, Leo, Saratoga, California 95070 (US); NAKAGAKI, Paul C., Saratoga, California 95070 (US); BANAIT, Narinder S., Saratoga, California 95070 (US)
(74) Representative: Cooley (UK) LLP
(86) International application number: PCT/US2016/012694
(87) International publication number: WO 2016/114993

(56) References cited:
- US-A1- 2011 171 193
- US-A1- 2013 251 787
- US-A1- 2014 135 358
- US-A1- 2014 163 086
- EDDA SPIEKERKOETTER ET AL: "CORRESPONDENCE Low-Dose FK506 (Tacrolimus) in End-Stage Pulmonary Arterial Hypertension", AMERICAN JOURNAL OF RESPIRATORY AND CRITICAL CARE MEDICIN, vol. 192, no. 2, 15 July 2015 (2015-07-15) , pages 254-257, XP055494613,
- EDDA SPIEKERKOETTER ET AL: "FK506 activates BMPR2, rescues endothelial dysfunction, and reverses pulmonary hypertension", JOURNAL OF CLINICAL INVESTIGATION, vol. 123, no. 8, 15 July 2013 (2013-07-15) , pages 3600-3613, XP055140950, ISSN: 0021-9738, DOI: 10.1172/JCI65592
- GREGORY P. STAR ET AL: "ALK2 and BMPR2 knockdown and endothelin-1 production by pulmonary microvascular endothelial cells", MICROVASCULAR RESEARCH., vol. 85, 1 January 2013 (2013-01-01), pages 46-53, XP055494614, US ISSN: 0026-2862, DOI: 10.1016/j.mvr.2012.10.012
- YAO, A.: 'Recent advances and future pe rspectives in therapeutic strategies for pulmonary arterial hypertension' JOURNAL OF CARDIOLOGY vol. 60, no. 5, 12 October 2012, pages 344 - 349, XP002734698
- SEFERIAN ET AL.: 'Therapies for pulmonary arterial hypertension: where are we today, where do we go tomorrow?' EUROPEAN RESPIRATORY REVIEW vol. 22, no. 129, 01 September 2013, pages 217 - 226, XP055464566 DOI: 0.1183/09059180.00001713

## Description

### FIELD OF INVENTION

The present disclosure relates to a combination comprising a stimulator of bone morphogenetic protein receptor type II (BMPR2) and one or more additional agents, for use in a method for the treatment or prevention of pulmonary hypertension, in particular pulmonary arterial hypertension.

### BACKGROUND

Pulmonary hypertension (PH) refers to a disease characterized by sustained elevations of pulmonary artery pressure. Generally, a patient having a mean pulmonary artery pressure equal to or greater than 25 mm Hg with a pulmonary capillary or left atrial pressure equal to or less than 15 mm Hg is characterized as having PH or as symptomatic of PH. These parameters may be measured in the subject at rest by right-heart catheterization.

The World Health Organization (WHO) has classified pulmonary hypertension into groups based on known causes. WHO group I includes patients with pulmonary arterial hypertension (PAH) including those patients with idiopathic PAH; familial PAH, and associated PAH, which is related to certain conditions including connective tissue diseases, congenital systemic-to-pulmonary-shunts, portal hypertension, HIV infection, drugs and toxins, glycogen storage disease, Gaucher's disease, hereditary hemorrhagic telangiectasia, hemoglobinopathies, myeloproliferative disorders, splenectomy, and others; PAH associated with significant venous or capillary involvement; and persistent pulmonary hypertension of the newborn. WHO group II includes patients with pulmonary venous hypertension. WHO group III includes patients with pulmonary hypertension associated with hypoxemia. WHO group IV includes patients with pulmonary hypertension due to chronic thrombotic disease, embolic disease or both. Finally, WHO group V includes patients with pulmonary hypertension due a variety of miscellaneous conditions.

The New York Heart Association (NYHA) further classifies pulmonary arterial hypertension into functional groups based on their exercise capacity and symptoms. NYHA functional class (FC) I includes patients with PAH without limitations of physical activity. FC II includes patients with PAH resulting in slight limitation of physical activity. FC III includes patients with PAH resulting in marked limitation in physical activity. FC IV includes patients with PAH that are unable to engage in physical activity without manifesting symptoms.

PAH is a serious, progressive and life-threatening disease of the pulmonary vasculature, characterized by profound vasoconstriction and an abnormal proliferation of smooth muscle cells in the walls of the pulmonary arteries. Severe constriction of the blood vessels in the lungs leads to very high pulmonary arterial pressures. These high pressures make it difficult for the heart to pump blood through the lungs to be oxygenated. Patients with PAH suffer from extreme shortness of breath as the heart struggles to pump against these high pressures. Patients with PAH typically develop significant increases in pulmonary vascular resistance (PVR) and sustained elevations in pulmonary artery pressure (PAP), which ultimately lead to right ventricular failure and death. Patients diagnosed with PAH have a poor prognosis and compromised quality of life, with a mean life expectancy of 2 to 5 years from the time of diagnosis if untreated.

PAH includes idiopathic pulmonary arterial hypertension; familial pulmonary arterial hypertension; pulmonary arterial hypertension in the setting of connective tissue diseases (e.g., localized cutaneous systemic sclerosis (CREST syndrome), diffuse scleroderma, systemic lupus erythematosus, mixed connective tissue disease, and other less common diseases), portal hypertension, congenital left-to-right intracardiac shunts, and infection with the human immunodeficiency virus); and persistent pulmonary hypertension of the newborn.

Current therapies for pulmonary hypertension are unsatisfactory. These typically involve calcium channel antagonists, prostacyclins, prostacyclin receptor (IP receptor) agonist, endothelin receptor antagonists, phosphodiesterase-5 (PDE5) inhibitors, and long-term anticoagulant therapy. However, each treatment has limitations and side effects. Importantly, the current therapeutic approaches mainly provide symptomatic relief and some improvement of prognosis. In addition, the current therapies have either undesired side effects or inconvenient drug administration routes.

US2014/0135358 describes FK506 (also known as tacrolimus or fujimycin) for use in treating pulmonary arterial hypertension associated with defective BMPR2 signaling. Atsushi Yao, Journal of Cardiology, 60:5, pp.344-349, 2012, refers to PAH drugs such as a receptor tyrosine kinase antagonist (imatinib), a soluble guanylate cyclase stimulator (riociguat), an oral analog of prostacyclin (selexipag), and a tissue targeting ERA (macitentan), and suggests that the discovery of efficacious combination therapies could lead to advances in the treatment of PAH. Consequently there is a long felt need for a new and combined medicament for the treatment of PAH, preferably employing lower doses of the active agents, which exhibits fewer or no adverse effects. (i.e., less toxicity) and a favorable profile in terms of effectiveness in patients in different stages of PAH.

### SUMMARY

The present invention is defined by the pending claim set. Any references to 'aspects' or 'the disclosure' relate to subject-matter that is not explicitly claimed and does not define the scope of the invention, which is defined by the claims. The disclosure provides compositions for use in a method of treating pulmonary hypertension, in particular pulmonary arterial hypertension.

In one aspect, the present disclosure describes a method of treating or preventing pulmonary hypertension in a patient in need thereof, the method comprising administering a therapeutically effective amount of a compound that increases BMPR2 signaling (BMPR2 activator) to the patient with pulmonary hypertension in combination with another active agent effective for treatment of the pulmonary hypertension condition or a condition related thereto. The subject can be a mammal, such as a human. The BMPR2 activator is tacrolimus or a pharmaceutically acceptable solvate or salt thereof, and the daily dose provides serum concentration of about 0.02 ng/mL to about 10 ng/mL. The second active agent comprises at least one drug selected from the group consisting of an endothelin receptor antagonist, a prostacyclin receptor agonist, prostanoid, a phosphodiesterase (PDE) inhibitor, a guanylate cyclase activator, an anti-inflammatory agent, a calcium channel blocker, a diuretic, an anticoagulant, oxygen and a combination thereof.

In another aspect, the present disclosure describes a method of treating or preventing pulmonary hypertension in a patient in need thereof, the method comprising administering a therapeutically effective amount of tacrolimus to the patient with pulmonary arterial hypertension in combination with another active agent effective for treatment of the pulmonary hypertension condition or a condition related thereto. The second active agent comprises at least one drug selected from the group consisting of an endothelin receptor antagonist, a prostacyclin receptor agonist, prostanoid, a phosphodiesterase (PDE) inhibitor, a guanylate cyclase activator, an anti-inflammatory agent, a calcium channel blocker, a diuretic, an anticoagulant, oxygen and a combination thereof. The PDE5 inhibitor can be avanafil, udenafil, or a pharmaceutically acceptable solvate or salt thereof.

In another aspect, the present disclosure describes a method of treating or preventing pulmonary hypertension in a patient in need thereof, the method comprising administering a therapeutically effective amount of a compound that increases BMPR2 signaling (BMPR2 activator) to the patient with pulmonary arterial hypertension. The BMPR2 activator is administered to improve exercise ability, delay clinical worsening, or combinations thereof.

### BRIEF DESCRIPTION OF DRAWINGS

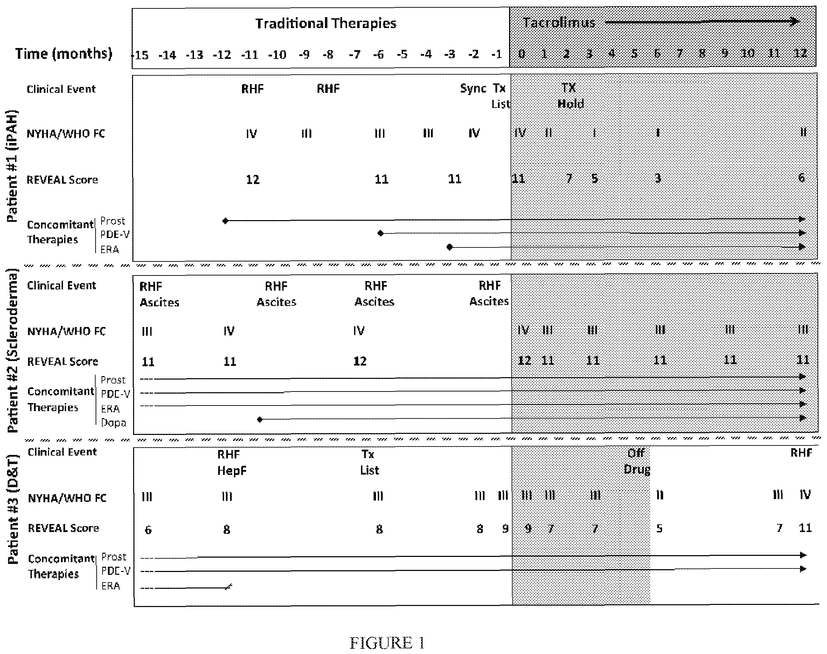

Figure 1 illustrates the clinical course of compassionate patients 1, 2 and 3 prior (clear) and after (shaded) initiation of tacrolimus. In the Figure, RHF means right heart failure, Sync means syncope, Tx List means patient placed on lung transplant list, Tx Hold means patient placed on hold on the lung transplant list, Prost means prostacyclin, Dopa means dopamine, D&T means drug and toxin induced PAH, iPAH means idiopathic PAH, and HepF means hepatic failure.

### DETAILED DESCRIPTION

### I. Definitions

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise. Definition of standard chemistry terms may be found in reference works, including Carey and Sundberg (2004) "Advanced Organic Chemistry 4rd Ed." Vols. A and B, Springer, New York. The practice of the present disclosure will employ, unless otherwise indicated, conventional methods of mass spectroscopy, protein chemistry, biochemistry, and pharmacology, within the skill of the art.

The term "modulator" means a molecule that interacts with a target. The interactions include, but are not limited to, agonist, antagonist, and the like, as defined herein.

The term "agonist" means a molecule such as a compound, a drug, an enzyme activator or a hormone that enhances the activity of another molecule or the activity of the target receptor.

The term "antagonist" means a molecule such as a compound, a drug, an enzyme inhibitor, or a hormone, that diminishes or prevents the action of another molecule or the activity of the target receptor.

The terms "effective amount" or "pharmaceutically effective amount" refer to a sufficient amount of the agent to provide the desired biological result without an unacceptable toxic effect. That result can be reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in a disease. An appropriate "effective" amount in any individual case may be determined by one of ordinary skill in the art using routine experimentation.

As used herein, the terms "treat" or "treatment" are used interchangeably and are meant to ameliorating the disease or disorder (i.e., arresting or reducing the development of the disease or at least one of the clinical symptoms thereof). In one aspect "treating" or "treatment" refers to ameliorating at least one symptoms of the disease. In another aspect, "treating" or "treatment" refers to inhibiting the disease or disorder, either physically (e.g., stabilization of a discernible symptom), physiologically, (e.g., stabilization of a physical parameter), or both.

By "pharmaceutically acceptable" or "pharmacologically acceptable" is meant a material which is not biologically or otherwise undesirable, i.e., the material may be administered to an individual without causing any undesirable biological effects or interacting in a deleterious manner with any of the components of the composition in which it is contained.

As used herein, the term "mammal subject" encompasses any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like.

The term "pharmaceutically acceptable salt" of a compound means a salt that is pharmaceutically acceptable and that possesses the desired pharmacological activity of the parent compound. Such salts, for example, include:
(1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1 -carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like;
(2) salts formed when an acidic proton present in the parent compound either is replaced by a metal ion, e.g., an alkali metal ion, an alkaline earth ion, or an aluminum ion; or coordinates with an organic base. Acceptable organic bases include ethanolamine, diethanolamine, triethanolamine, tromethamine, N-methylglucamine, and the like. Acceptable inorganic bases include aluminum hydroxide, calcium hydroxide, potassium hydroxide, sodium carbonate, sodium hydroxide, and the like. It should be understood that a reference to a pharmaceutically acceptable salt includes the solvent addition forms or crystal forms thereof, particularly solvates or polymorphs. Solvates contain either stoichiometric or non-stoichiometric amounts of a solvent, and are often formed during the process of crystallization. Hydrates are formed when the solvent is water, or alcoholates are formed when the solvent is alcohol. Polymorphs include the different crystal packing arrangements of the same elemental composition of a compound. Polymorphs usually have different X-ray diffraction patterns, infrared spectra, melting points, density, hardness, crystal shape, optical and electrical properties, stability, and solubility. Various factors such as the recrystallization solvent, rate of crystallization, and storage temperature may cause a single crystal form to dominate.

The term "optional" or "optionally" means that the subsequently described event or circumstance may or may not occur, and that the description includes instances where the event or circumstance occurs and instances where it does not.

### II. DESCRIPTION OF THE INVENTION

The combinations of the present disclosure increase BMPR2 pathway signaling. In certain aspects, the combinations of the present disclosure have reduced toxicity and fewer adverse side effects.

Therefore, in one aspect, the combinations of the present disclosure can be useful for the prevention or treatment of pulmonary hypertension (PH), in particular pulmonary arterial hypertension (PAH). Compounds that increase the signaling of the BMPR2 pathway can further be combined with other compounds that increase vasodilation such as compounds that target endothelin (bosentan (Tracleer^{®}), macetentan (Opsumit^{®}), and ambrisentan (Letairis^{®})), nitric oxide/PDE-5 (sildenafil (Revatio^{®}), tadalafil (Adcirca^{®}), avanafil, lodenafil, mirodenafil, udenafil, and zaprinast), prostacyclin (treprostinil (Remodulin^{®}, Tyvaso^{®}, or Orenitram ^{®}) and epoprostenol (Flolan^{®})), prostacyclin receptor agonists (selexipag (Uptravi^{®}), and APD811), soluble guanylate cyclase (adempas (Riociguat^{®})), apoptosis signal-regulating kinase 1 (ASK 1) inhibitor (GSK-4997, GSK 444217), and the like. Thus, the combined compounds can become more effective agents for the treatment of PAH, and may provide synergistic results from the combined use of the compounds that increase the signaling of the BMPR2 pathway with compounds that target other pathways. The combinations described in detail herein can provide a safe pharmaceutical agent for combination therapies in humans with few side effects and efficacious treatment results.

The currently approved targeted PAH therapeutics have been developed to address the mechanistic pathways of increased vasoconstriction in PAH, however, treatment strategies to reverse vascular remodeling have been lacking.

Germline mutations causing loss of BMPR2 function are found in >80% of familial and approximately 20% of sporadic cases of IPAH. Acquired somatic chromosomal abnormalities in the BMPR2 signaling pathway have also been described. The low penetrance of pulmonary arterial hypertension (PAH) found in non-affected family members with a BMPR2 mutation has been attributed to a higher level of BMPR2 expression from the normal allele. In addition, patients with IPAH without a BMPR2 mutation or with PAH associated with other conditions have reduced expression of BMPR2 in pulmonary arteries. Furthermore, estrogen can reduce BMPR2 expression, perhaps explaining the propensity of females to develop PAH. IL-6, a cytokine increased in the blood of patients with IPAH, can reduce BMPR2 expression via a STAT3-miR17/92-mediated mechanism. Furthermore, patients with a BMPR2 mutation have worse pulmonary vascular remodeling. The importance of BMPR2 dysfunction in PAH is supported by studies in transgenic mice. Mice with deletion of BMPR2 in endothelial cells (ECs) develop PAH, as do mice expressing a dominant-negative *Bmpr2* gene after birth in vascular smooth muscle cells (SMC). Reduced BMPR2 expression also occurs in monocrotaline and chronic hypoxic rat models of PAH, and delivery of BMPR2 by intravenous gene therapy attenuates the disease in both models. Moreover reconstitution of athymic rats with regulatory T cells also prevents PAH resulting from blockade of the vascular endothelial growth factor (VEGF) receptor, coincident with an increase in BMPR2 expression in ECs.

The BMPR2 pathway is thus a critically important pathway that is reduced in PAH and, therefore, increasing BMPR2 signaling in patients with PAH can prevent or reverse disease.

A C2C12 mouse myoblastoma reporter cells line where the BRE (BMP Response Element) from the Id1 promoter (a main downstream target of BMP signaling) was linked to luciferase (BRE-Luc) was used to identify compounds that increase BMPR2 signaling. Thus, activation of the BMP pathway was measured by luminescence. A high throughput screen of a library containing greater than 3,600 compounds was used. Tacrolimus, rapamycin, and cyclosporin were identified as compounds that increased BMPR2 signaling.

A compound that increases BMPR2 signaling or a pharmaceutically acceptable solvate, salt, or prodrug thereof can be administered to a patient for the treatment or prevention of PAH. Treatment or prevention of PAH as used herein encompasses one or more of the following:
(a) adjustment of one or more hemodynamic parameters towards a more normal level, for example lowering mean PAP or PVR, or raising PCWP or LVEDP, versus baseline;
(b) improvement of pulmonary function versus baseline, for example increasing exercise capacity, illustratively as measured in a test of 6-minute walking distance (6MWD), or lowering Borg dyspnea index (BDI);
(c) improvement of one or more quality of life parameters versus baseline, for example an increase in score on at least one of the SF-36^{™} health survey functional scales;
(d) general improvement versus baseline in the severity of the condition, for example by movement to a lower WHO functional class;
(e) improvement of clinical outcome following a period of treatment, versus expectation in absence of treatment (e.g., in a clinical trial setting, as measured by comparison with placebo), including improved prognosis, extending time to or lowering probability of clinical worsening, extending quality of life (e.g., delaying progression to a higher WHO functional class or slowing decline in one or more quality of life parameters such as SF-36^{™} health survey parameters), and/or increasing longevity; and/or
(f) adjustment towards a more normal level of one or more molecular markers that can be predictive of clinical outcome, such as plasma concentrations of bone morphogenetic protein (BMP), cardiac troponin T (cTnT), NT-proBNP, or B-type natriuretic peptide (BNP)).

The compound to increase BMPR2 signaling can be administered in a therapeutically effective amount sufficient to provide any one or more of the effects mentioned above. Preferably the amount administered does not exceed an amount causing an unacceptable degree of adverse side effects. The therapeutically effective amount can vary depending on the compound, the particular pulmonary hypertension condition to be treated, the severity of the condition, body weight and other parameters of the individual subject, and can be readily established without undue experimentation by the physician or clinician based on the disclosure herein. Typically, a therapeutically effective amount will be found in the range of about 1 to about 25 mg/day, for example about 2 to about 15 mg/day, about 2.5 to about 10 mg/day, or about 2.5, about 3, about 3.5, about 4, about 4.5, about 5, about 6, about 7, about 8, about 9 or about 10 mg/day. The therapeutically effective amount can be administered each day, for example in individual doses administered once, twice, or three or more times a day. The therapeutically effective amount can be administered once each day, once every other day, or once every third day.

For example, if the compound to increase BMPR2 signaling is tacrolimus or a pharmaceutically acceptable solvate or salt thereof, tacrolimus can be administered at a dose and regimen that provides tacrolimus serum concentration of about 0.05 ng/ml to about 10 ng/ml, such as about 0.1 ng/ml to about 0.5 ng/ml, about 0.15 ng/ml to about 0.3 ng/ml or about 0.1-0.2 ng/ml. In part because tacrolimus is metabolized by the cytochrome P450 system, the exact dosing may vary between patients. Tacrolimus can be administered once, twice, or three or more times a day. In one aspect, the goal is to reach a serum level of about 0.2 ng/ml. In this case, an initial dose of 0.001 mg/kg day to 0.01 mg/kg day (e.g., 0.002 mg kg /day to 0.05 mg/kg/day may be sufficient, and the does can be up-titrated according to the measured tacrolimus serum level. In particular cases, the tacrolimus may reach a serum concentration as low as 0.1-0.2 ng/ml (e.g., 0.10 to 0.12, 0.12 to 0.14, 0.14 to 0.16, 0.16 to 0.18 or 0.18 to 0.20), however serum a concentration in the range of 0.2 to 2 ng/ml, e.g., 0.2, 0.5, 1 and 2 ng/ml may be acceptable. In particular cases, tacrolimus can reach a serum concentration of <1.0, 1.5-2.5, or 3-5 ng/ml.

The active agent to increase BMPR2 signaling can be administered in monotherapy. Alternatively, the compound to increase BMPR2 signaling can be administered in combination therapy with one or more other active agent effective for the treatment of the pulmonary hypertension condition or a condition related thereto. When a second or more active agent is administered concomitantly, one of skill in the art can readily identify a suitable dose for any particular second active agent from publicly available information in printed or electronic form, for example on the internet. Illustratively and without limitation, the active agent to increase BMPR2 signaling can be administered with a second active agent comprising at least one drug selected from the group consisting of prostanoids, phosphodiesterase inhibitors, especially phosphodiesterase-5 (PDE5) inhibitors, endothelin receptor antagonists (ERAs), prostacyclin receptor (IP receptor) agonist, soluble guanylate cyclase stimulator, calcium channel blockers, ASK-1 inhibitor, an inhibitor of proliferative signaling, an inhibitor of inflammatory signaling, diuretics, anticoagulants, nitric oxide, oxygen and combinations thereof.

Examples of drugs useful in combination therapy are classified and presented in several lists below. Some drugs are active at more than one target; accordingly certain drugs may appear in more than one list. Use of any listed drug in a combination is contemplated herein, independently of its mode of action.

A suitable prostanoid can be illustratively selected from the following list: beraprost, cicaprost, epoprostenol, iloprost, NS-304, PGE₁ prostacyclin, and treprostinil.

A suitable PDE5 inhibitor can illustratively be selected from the following list: sildenafil, tadalafil, vardenafil, avanafil, lodenafil, mirodenafil, udenafil, and zaprinast.

A suitable ERA other than ambrisentan can illustratively be selected from the following list: atrasentan, ambrisentan, BMS 193884, bosentan, macitentan, CI-1020, darusentan, S-0139 SB-209670, sitaxsentan, TA-0201, tarasentan, TBC-3711, VML-588, and ZD-1611.

A suitable prostacyclin (IP) receptor agonists can illustratively be selected from selexipag (Uptravi^{®}) or APD811.

A suitable ASK-1 inhibitor can illustratively be selected from GSK-4997 or GSK 444217.

A suitable inhibitor of proliferative signaling can illustratively be selected from imatinib or nilotinib.

A suitable inhibitor of inflammatory signaling can illustratively be selected from ubenimex or bardoxolone methyl.

A suitable calcium channel blocker can illustratively be selected from the following list: Aryklalkylamines: bepridil, clentiazem, diltiazem, fendiline, gallopamil, mibefradil, prenylamine, semotiadil, terodiline, and verapamil; Dihydropyridine, derivatives: amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, and NZ 105; Piperazine derivatives: cinnarizine, dotarizine, flunarizine, lidoflazine, and lomerizine; and Unclassified: bencyclane, etafenone, fantofarone, monatepil, perhexiline. Particularly suitable calcium channel blockers include amlodipine, diltiazem, felodipine, isradipine, nicardipine, nifedipine, nisoldipine, verapamil and combinations thereof.

A suitable diuretic can illustratively be selected from the following list: Organomercurials: chlormerodrin, chlorothiazide, chlorthalidone, meralluride, mercaptomerin, sodium mercumatilin, sodium mercurous, and chloride mersalyl; Purines: pamabrom, protheobromine, and theobromine; Steroids: canrenone, oleandrin, and spironolactone; Sulfonamide derivatives: acetazolamide, ambuside, azosemide, bumetanide, butazolamide, chloraminophenamide, clofenamide, clopamide, clorexolone, disulfamide, ethoxzolamide, furosemide, mefruside, methazolamide, piretanide, torsemide, tripamide, and xipamide; Thiazides and analogs: althiazide, bendroflumethiazide, benzthiazide, benzylhydrochlorothiazide, buthiazide, chlorthalidone, cyclopenthiazide, cyclothiazide, ethiazide, fenquizone, hydrochlorothiazide, hydroflumethiazide, indapamide, methyclothiazide, metolazone, paraflutizide, polythiazide, quinethazone, teclothiazide, and trichlormethiazide; Uracils: aminometradine; Unclassified: amiloride, Biogen BG 9719, chlorazani1, ethacrynic acid, etozolin, isosorbide, Kiowa Hakko KW 3902, mannitol, muzolimine, perhexiline, Sanofi-Aventis SR 121463, ticrynafen, triamterene, and urea. In some aspects, the diuretic if present comprises a thiazide or loop diuretic. Thiazide diuretics are generally not preferred where the patient has a complicating condition such as diabetes or chronic kidney disease, and in such situations a loop diuretic can be a better choice. Particularly suitable thiazide diuretics include chlorothiazide, chlorthalidone, hydrochlorothiazide, indapamide, metolazone, polythiazide and combinations thereof. Particularly suitable loop diuretics include bumetanide, furosemide, torsemide and combinations thereof.

A suitable anticoagulant can illustratively be selected from the following list: acenocoumarol, ancrod, anisindione, bromindione, clorindione, coumetarol, cyclocumarol, dextran sulfate, sodium dicumarol, diphenadione, ethyl biscoumacetate, ethylidene dicoumarol, fluindione, heparin, hirudin, lyapolate, sodium pentosan, polysulfate phenindione, phenprocoumon, phosvitin, picotamide, tioclomarol, and warfarin.

Where the pulmonary hypertension condition is associated with an underlying disease (for example CTD, HIV infection, COPD or ILD), the active agent to increase BMPR2 signaling can optionally be administered in combination therapy with one or more drugs targeting the underlying condition.

When the active agent to increase BMPR2 signaling is used in combination therapy with one or more drugs, the active agent and at least one drug can be administered at different times or at about the same time (at exactly the same time or directly one after the other in any order). The active agent and the second active drug can be formulated in one dosage form as a fixed-dose combination for administration at the same time, or in two or more separate dosage forms for administration at the same or different times.

The dosage of the additional drugs can be obtained from readily available sources, such as, for example, the product inserts. For example, if the other drug is bosentan, treatment is initiated at 62.5 mg twice a day for 4 weeks, and then the dosage is increased to 125 mg twice daily. The recommended dosage of macitentan is 10 mg once a day, while the initial dosage of ambrisentan is 5 mg once a day that can be increased to 10 mg once a day. The recommended dosage of sildenafil is 5 mg or 20 mg three times a day about 4-6 hours apart, while the dosage of tadalafil is 40 mg once a day. The recommended initial dosage of selexipeg is 200 mcg twice a day that is increased in increments of 200 mcg to the highest tolerated dose, while the recommended initial dosage of oral treprostinil is 0.25 mg twice a day that is increased until optimal clinical response is achieved.

Separate dosage forms can optionally be co-packaged, for example in a single container or in a plurality of containers within a single outer package, or co-presented in separate packaging ("common presentation"). As an example of co-packaging or common presentation, a kit is contemplated comprising, in separate containers, active agent to increase BMPR2 signaling and at least one drug useful in combination with the active agent. In another example, the active agent and the at least one drug useful in combination therapy with the active agent are separately packaged and available for sale independently of one another, but are co-marketed or co-promoted for use according to the disclosure. The separate dosage forms can also be presented to a patient separately and independently, for use according to the disclosure.

### III. ADDITIVITY / SYNERGY

In one aspect of the disclosure, the administration of an active agent to increase BMPR2 signaling and a second active agent to a patient results in additive or synergistic therapeutic effects. The term "additive" refers to the expected magnitude of therapeutic effect that results when one therapeutic agent is combined with another therapeutic agent. The term "synergistic" as used herein refers to a therapeutic combination which is more effective than the additive effects of the two or more single agents. Synergism is defined herein as a more than expected additive effect, and antagonism as a less than expected additive effect as proposed by Cho and Talalay J. Biol. Chem. 252:6438-6442 (1977).

A determination of a synergistic interaction between an active agent to increase BMPR2 signaling and a second active agent can be based on results analyzed using the Chou and Talalay combination method and Dose-Effect Analysis with CalcuSyn software in order to obtain a Combination Index (Chou and Talalay, Adv. Enzyme Regul. 22:27-55 (1984)). The combinations provided by this disclosure can be evaluated in several assay systems, and the data can be analyzed utilizing a standard program for quantifying synergism, additivism, and antagonism among agents used in the therapeutic agents.

Combination Index (CI) values less than 0.8 indicates synergy, values greater than 1.2 indicate antagonism and values between 0.8 to 1.2 indicate additive effects. The combination therapy can provide "synergy" and prove "synergistic," i.e., the effect achieved when the active ingredients used together is greater than the sum of the effects that results from using the compounds separately.

A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds are administered or delivered sequentially. In general, during alternation therapy, an effective dosage of each active ingredient is administered sequentially, i.e., serially, whereas in combination therapy, effective dosages of two or more active ingredients are administered together.

The amount of composition disclosed herein to be administered to a patient to be effective (i.e. to provide exposures of an active agent sufficient to be effective in the treatment or prevention of PAH) will depend upon the bioavailability of the particular composition, the amount and potency of the active agents present in the composition, as well as other factors, such as the species, age, weight, sex, and condition of the patient, manner of administration and judgment of the prescribing physician.

The ratio of the active agent to increase BMPR2 signaling and a second active agent are selected such that CI is less than about 1.2, preferably less than about 0.9, more preferably less than about 0.8, most preferably less than about 0.75. In another aspect, the CI for the combination is between about 0 and 0.9, preferably between about 0.4 to about 0.8, more preferably between about 0.5 and 0.75, most preferably between about 0.55 and 0.7.

### IV. Formulations

The compounds described above are preferably used to prepare a medicament, such as by formulation into pharmaceutical compositions for administration to a subject using techniques generally known in the art. A summary of such pharmaceutical compositions may be found, for example, in Remington's Pharmaceutical Sciences, Mack Publishing Co., Easton, PA. The compounds of the disclosure can be used singly or as components of mixtures. Preferred forms of the compounds are those for systemic administration as well as those for topical or transdermal administration. Formulations designed for timed release are also with the scope of the disclosure. Formulation in unit dosage form is also preferred for the practice of the disclosure.

In unit dosage form, the formulation is divided into unit doses containing appropriate quantities of one or more compound. The unit dosage may be in the form of a package containing discrete quantities of the formulation. Non-limiting examples are packeted tablets or capsules, and powders in vials or ampoules.

The compounds of the disclosure may be labeled isotopically (e.g. with a radioisotope) or by another other means, including, but not limited to, the use of chromophores or fluorescent moieties, bioluminescent labels, or chemiluminescent labels. The compositions may be in conventional forms, either as liquid solutions or suspensions, solid forms suitable for solution or suspension in a liquid prior to use, or as emulsions. Suitable excipients or carriers are, for example, water, saline, dextrose, glycerol, alcohols, aloe vera gel, allantoin, glycerin, vitamin A and E oils, mineral oil, propylene glycol, PPG-2 myristyl propionate, and the like. Of course, these compositions may also contain minor amounts of nontoxic, auxiliary substances, such as wetting or emulsifying agents, pH buffering agents, and so forth.

Methods for the preparation of compositions comprising the compounds of the disclosure include formulating the derivatives with one or more inert, pharmaceutically acceptable carriers to form either a solid or liquid. Solid compositions include, but are not limited to, powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein.

A carrier of the disclosure can be one or more substances which also serve to act as a diluent, flavoring agent, solubilizer, lubricant, suspending agent, binder, or tablet disintegrating agent. A carrier can also be an encapsulating material.

In powder forms of the disclosure's compositions, the carrier is preferably a finely divided solid in powder form which is interdispersed as a mixture with a finely divided powder form of one or more compound. In tablet forms of the compositions, one or more compounds is intermixed with a carrier with appropriate binding properties in suitable proportions followed by compaction into the shape and size desired. Powder and tablet form compositions preferably contain between about 5 to about 70% by weight of one or more compound. Carriers that may be used in the practice of the disclosure include, but are not limited to, magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The compounds of the disclosure may also be encapsulated or microencapsulated by an encapsulating material, which may thus serve as a carrier, to provide a capsule in which the derivatives, with or without other carriers, is surrounded by the encapsulating material. In an analogous manner, cachets comprising one or more compounds are also provided by the instant disclosure. Tablet, powder, capsule, and cachet forms of the disclosure can be formulated as single or unit dosage forms suitable for administration, optionally conducted orally.

If administered orally, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

Formulations for parenteral administration may be in the form of aqueous or non-aqueous sterile injection solutions or suspensions. These solutions and suspensions can be prepared from sterile powders or granules having one or more of the carriers or diluents mentioned for use in the formulations for oral administration. The compounds can be dissolved or suitably emulsified in water, polyethylene glycol, propylene glycol, ethanol, corn oil, cottonseed oil, peanut oil, sesame oil, benzyl alcohol, sodium chloride, and/or various buffers. Other adjuvants and modes of administration are widely known in the pharmaceutical art.

For oral administration, the pharmaceutical composition can be in the form of, for example, a tablet, capsule, a soft gelatin (softgel) capsule, a hard gelatin capsule, suspension or liquid. The pharmaceutical composition is preferably made in the form of a dosage unit containing a particular amount of the active ingredient. Examples of such dosage units are tablets or softgel capsules. The active ingredient can also be administered by injection as a composition wherein, for example, saline, dextrose or water can be used as a suitable carrier.

Soft gelatin capsules can be prepared in which capsules contain a mixture of a BMPR2 activator and at least one other active compound, and oleaginous and/or non-aqueous, and/or water miscible solvents such as polyethylene glycol and the like. Hydrophilic solvents compatible with softgel capsules can include PEG400, PEG800, ethanol, glycerin, PPG, polysorbates, povidone (PVP), and the like containing up to about 5-8% water. The softgel capsules can optionally contain a buffer, a co-solvent, or a nucleophile. Hard gelatin capsules can contain mixtures of BMPR2 activator and at least one other active compound in combination with a solid, pulverulent carrier, such as, for example, lactose, saccharose, sorbitol, mannitol, potato starch, corn starch, amylopectin, cellulose derivatives, or gelatin.

In suppository forms of the compositions, a low-melting wax such as, but not limited to, a mixture of fatty acid glycerides, optionally in combination with cocoa butter is first melted. One or more compounds are then dispersed into the melted material by, as a non-limiting example, stirring. The non-solid mixture is then placed into molds as desired and allowed to cool and solidify.

Non-limiting compositions in liquid form include solutions suitable for oral or parenteral administration, as well as suspensions and emulsions suitable for oral administration. Sterile aqueous based solutions of one or more compounds, optionally in the presence of an agent to increase solubility of the derivative(s), are also provided. Non-limiting examples of sterile solutions include those comprising water, ethanol, and/or propylene glycol in forms suitable for parenteral administration. A sterile solution of the disclosure may be prepared by dissolving one or more compounds in a desired solvent followed by sterilization, such as by filtration through a sterilizing membrane filter as a non-limiting example. In another aspect, one or more compounds are dissolved into a previously sterilized solvent under sterile conditions.

A water based solution suitable for oral administration can be prepared by dissolving one or more compounds in water and adding suitable flavoring agents, coloring agents, stabilizers, and thickening agents as desired. Water based suspensions for oral use can be made by dispersing one or more compounds in water together with a viscous material such as, but not limited to, natural or synthetic gums, resins, methyl cellulose, sodium carboxymethyl cellulose, and other suspending agents known to the pharmaceutical field.

Pulmonary administration can be achieved by inhalation or by the introduction of a delivery device into the pulmonary system, e.g., by introducing a delivery device which can dispense (wet or dry) the pharmaceutical composition. The BMPR2 activator or its combination with at least one other active compound can be provided in a dispenser which delivers the composition in a form sufficiently small such that it can be inhaled. The BMPR2 activator or its combination can be provided in measured doses, in a dispenser that delivers a metered dose, or a dry powder inhaler.

In therapeutic use, the compounds of the disclosure (BMPR2 activators and/or one or more another active agents) are each administered to a subject at a dosage level of from about 0.05-8, 0.05-80, 0.5-8, or 0.5-80 mg/kg, of body weight per day. For example, in a human subject of approximately 70 kg, this is a dosage of from 4 mg to 600 mg per day. Such dosages, however, may be altered depending on a number of variables, not limited to the activity of the compound used, the condition to be treated, the mode of administration, the requirements of the individual subject, the severity of the condition being treated, and the judgment of the practitioner.

The foregoing ranges are merely suggestive, as the number of variables in regard to an individual treatment regime is large, and considerable excursions from these recommended values are not uncommon.

### V. Methods of use

A compound of the disclosure can be administered to a subject upon determination of the subject as having pulmonary hypertension, in particular pulmonary arterial hypertension, or unwanted condition that would benefit by treatment with said derivative. The determination can be made by medical or clinical personnel as part of a diagnosis of a disease or condition in a subject.

For administration to non-human animals, the drug or a pharmaceutical composition containing the drug may also be added to the animal feed or drinking water. It will be convenient to formulate animal feed and drinking water products with a predetermined dose of the drug so that the animal takes in an appropriate quantity of the drug along with its diet. It will also be convenient to add a premix containing the drug to the feed or drinking water approximately immediately prior to consumption by the animal.

### Kits/Articles of manufacture

For use in the therapeutic applications described herein, kits and articles of manufacture are also within the scope of the disclosure. Such kits can comprise a carrier, package, or container that is compartmentalized to receive one or more containers such as vials, tubes, and the like, each of the container(s) comprising one of the separate elements to be used in a method of the disclosure. Suitable containers include, for example, bottles, vials, syringes, and test tubes. The containers can be formed from a variety of materials such as glass or plastic.

For example, the container(s) can comprise one or more compounds of the disclosure, optionally in a composition or in combination with another agent as disclosed herein. The container(s) optionally have a sterile access port (for example the container can be an intravenous solution bag or a vial having a stopper pierceable by a hypodermic injection needle). Such kits optionally comprising a compound with an identifying description or label or instructions relating to its use in the methods of the present disclosure.

A kit of the disclosure will typically may comprise one or more additional containers, each with one or more of various materials (such as reagents, optionally in concentrated form, and/or devices) desirable from a commercial and user standpoint for use of a compound of the disclosure. Non-limiting examples of such materials include, but not limited to, buffers, diluents, filters, needles, syringes; carrier, package, container, vial and/or tube labels listing contents and/or instructions for use, and package inserts with instructions for use. A set of instructions will also typically be included.

A label can be on or associated with the container. A label can be on a container when letters, numbers or other characters forming the label are attached, molded or etched into the container itself; a label can be associated with a container when it is present within a receptacle or carrier that also holds the container, e.g., as a package insert. A label can be used to indicate that the contents are to be used for a specific therapeutic application. The label can also indicate directions for use of the contents, such as in the methods described herein.

The terms "kit" and "article of manufacture" may be used as synonyms.
disclosure.

### EXAMPLES

### EXAMPLE 1

### Clinical Study

Tacrolimus was selected as the agent for increasing BMPR2 signaling. A phase IIA trial to assess the safety and tolerability of different doses tacrolimus (goal blood level < 2 ng/ml, 2-3 ng/ml, 3-5 ng/ml) vs Placebo was conducted (Clinical Trials Identifier: NCT01647945). The secondary endpoints were change in clinical worsening, change in 6-min walk distance, NT-pro BNP, Uric Acid, and novel echo parameters. The phase IIA trial was a single center trial with 23 patients. This study was open to male or female subjects, 18-70 years of age, with pulmonary hypertension. The Inclusion and Exclusion criteria used are below:
Inclusion Criteria
   1. Age ≥ 18 and < 70 years
   2. Diagnosis of WHO Group I Pulmonary Arterial Hypertension (PAH) (Idiopathic (I)PAH, Heritable PAH (including Hereditary Hemorrhagic Telangiectasia), Associated (A)PAH (including collagen vascular disorders, drugs+toxins exposure, congenital heart disease, and portopulmonary disease).
   3. Stable on active PAH treatment including any prostacycline or phosphodiesterase inhibitors and the endothelin antagonist Ambrisentan alone or in combination (stability defined as: <10% change in 6MWD, no change in NYHA class, no hospitalization or addition of PAH therapy for at least 3 months).
   4. Previous Right Heart Catheterization (RHC) that documented:
      a. Mean PAP ≥ 25 mmHg.
      b. Pulmonary capillary wedge pressure < 15 mmHg.
      c. Pulmonary Vascular Resistance ≥ 3.0 Wood units or 240 dynes/sec/cm5
   5. All NYHA/WHO functional classes.
   6. Willingness of female subjects to use birth control, or be post-menopausal, or status post hysterectomy.
Exclusion Criteria:
   1. WHO Group II - V Pulmonary Hypertension.
   2. Current or prior experimental PAH treatments within the last 6 months (including but not limited to tyrosine kinase inhibitors, rho-kinase inhibitors, or cGMP modulators).
   3. Current active treatment with the dual endothelin receptor antagonist bosentan.
   4. Total lung Capacity (TLC) < 60% predicted; if TLC b/w 60 and 70% predicted, high resolution computed tomography must be available to exclude significant interstitial lung disease.
   5. Forced expiratory volume (FEV1) / Forced Vital Capacity (FVC) < 70% predicted and FEV1 < 60% predicted
   6. Significant left-sided heart disease (based on screening Echocardiogram):
      a. Significant aortic or mitral valve disease
      b. Diastolic dysfunction ≥ Grade II
      c. Left Ventricle (LV) systolic function < 45%
      d. Pericardial constriction
      e. Restrictive cardiomyopathy
      f. Significant coronary disease with demonstrable ischemia.
   7. Chronic renal insufficiency defined as an estimated creatinine clearance < 30 ml/min (by Modification of diet in renal disease (MDRD) equation).
   8. Current atrial arrhythmias not under optimal control.
   9. Uncontrolled systemic hypertension: SBP > 160 mm or DBP > 100mm
   10. Severe hypotension: SBP < 80 mmHg.
   11. Pregnant or breast-feeding.
   12. Psychiatric, addictive, or other disorder that compromises patient's ability to provide informed consent, to follow study protocol, and adhere to treatment instructions.
   13. Active cyclosporine use.
   14. Known allergy or hypersensitivity to tacrolimus.
   15. Planned initiation of cardiac or pulmonary rehabilitation during period of study.
   16. Human Immunodeficiency Virus infection.
   17. Moderate to severe hepatic dysfunction with a Child Pugh score >10.
   18. Hyperkalemia defined as Potassium > 5.1 mEq/L at screening.
   19. Known active infection requiring antibiotic, antifungal, or antiviral therapies.
   20. Co-morbid conditions that would impair a patient's exercise performance and ability to assess WHO functional class, including but not limited to chronic low-back pain or peripheral musculoskeletal problems.

### Objectives

This was a Phase II, randomized, double-blind, placebo-controlled trial, with one primary and two secondary specific aims. Specific aims 1 examines the safety and tolerability of tacrolimus in patients with PAH while specific aims 2 & 3 evaluate the effect of tacrolimus on clinical worsening (#2) and clinical markers such as exercise tolerance and disease biomarkers (#3). Patients in this protocol may be concurrently treated with other PAH therapies.

### Results

The results showed that tacrolimus was well tolerated at all tested doses, there were no drug-related serious adverse events, there were no incidences of hypertension or cardiovascular events, and biomarker data indicated an increase in BMPR2 signaling.

A sub-group analysis was performed on patients that were on multiple drugs for effect of the combination therapy. The changes in 6 minute walk distance (6MWD) and the level of NT-proBNP (pg/mL) after 16 weeks of therapy are shown in the table below:

| | Placebo | Tacrolimus and PDE5 inhibitor | Tacrolimus, PDE5, and ERA | Tacrolimus, PDE5, ERA, and prostacyclin |
|---|---|---|---|---|
| Change 6MWD | 4.07% | 16.0% | 9.5% | 0.2% |
| Change NT-ProBNP | -18.75% | -51.1% | -26.0% | -22.7% |

The data show that treatment of patients with tacrolimus improves the pulmonary function of the patients as measured by an increase in the 6MWD and adjusts the molecular biomarkers that can be predictive of clinical outcome towards a more normal level as measured by a very significant decrease in the NT-proBNP levels. Thus, the use of an agent that increases the BMPR2 signaling is efficacious for treating PAH.

### EXAMPLE 2

### Compassionate Use Study

Three PAH patients that did not meet the inclusion criteria for the Phase IIA clinical trial as they showed a continuous worsening of their PAH with regular hospital admissions due to New York Heart Association (NYHA) Class IV symptoms, were treated with tacrolimus with a goal blood level of 1.5 -2.5 ng/mL. The clinical parameters utilized were: NYHA functional class, six-minute walking distance (6MWD), serologic biomarkers (such as NT-pro BNP, a biomarker for heart failure), hospital admissions as well as standard and protocolized cardiac magnetic resonance imaging (cMRI) interpreted by an expert blinded to patient and date were used as clinical parameters.

**Patient #1:** A 36-year-old historically athletic female presented with progressive dyspnea on exertion and recent syncope consistent with NYHA Class IV symptoms. Echocardiography showed a moderately enlarged right ventricle (RV) with estimated RV systolic pressure of 100 mmHg. A right heart catheterization (RHC) demonstrated severe PAH: mean right atrial pressure (mRAP) 10 mmHg, mean pulmonary artery pressure (mPAP) 61 mmHg, pulmonary arterial wedge pressure (PAWP) 6 mmHg, cardiac output (CO) 2.1 L/min and pulmonary vascular resistance (PVR) 26.7 WU. The diagnostic work-up confirmed IPAH. Her baseline 6MWD was 365 meters consistent with substantial exercise limitation. The patient was admitted and initiated on intravenous epoprostenol. Despite initial improvement, she required rapid up titration of epoprostenol, and had recurrent hospitalizations for RV failure necessitating addition of PAH therapies, sildenafil and ambrisentan (**Figure 1**). Despite achieving a 6MWD of 515 meters on triple therapy (epoprostenol 41 ng/kg/min, sildenafil 30 mg tid, and ambrisentan 10 mg qd), the patient continued to report NYHA Class III/IV symptoms. Her NT-pro BNP was elevated to 1,202 pg/mL and she was referred for lung transplantation. At the time of transplant listing, the patient's Registry to Evaluate Early And Long-term PAH Disease Management (REVEAL) risk score was 11, stratifying her as high risk with a potential 1-year mortality of 15-30%^{3,13}. At that time she was offered compassionate oral treatment with tacrolimus. The goal was to achieve trough tacrolimus blood level of 1.5-2.5 ng/mL and there was no further increase in her PAH-specific therapies.

Within 1 month of FK-506 (tacrolimus) initiation, patient #1 reported substantial improvement in symptoms and exercise capacity (**Figure 1**). Within 2 months she was placed on a status 7 (hold) for transplantation by the Stanford Heart and Lung Transplant team. After 3 months of treatment, she had improvement in 6MWD by approximately 100 meters, reduction of symptoms to NYHA Class I level, and a >50% lowering of her NT-proBNP (678 pg/mL). cMRI data at baseline, 3 and 6 months showed stable RV ejection fraction (RVEF), RV end-diastolic volume index (RVEDVi), along with increased RV stroke volume index (RVSVi) and cardiac index (CI). Improvement in these parameters was further reflected in a reduction of the REVEAL risk score to 3 (range 3-6), placing her in the low risk category (**Figure 1**). While the 12 months prior to starting FK-506 were characterized by 3 hospitalizations for RV failure, the subsequent 12 months after initiation of this therapy were free of any PAH associated hospitalizations. The patient declined follow up RHC citing stable clinical symptoms.

**Patient #2:** This patient is a 50-year-old female with end-stage systemic sclerosis associated PAH on intravenous treprostinil 111 ng/kg/min, sildenafil 60 mg tid, ambrisentan 10 mg qd, as well as intravenous dopamine infusion at 5 mcg/kg/min for end-stage RV failure and hypotension. A RHC on the above medications, approximately 1 year prior to initiation of FK-506, demonstrated severe PAH as evidenced by mRAP 12 mmHg, mPAP 51 mmHg, PAWP 8 mmHg, CO 2.7 L/min, mixed-venous oxygen saturation (SVO2) 41% and PVR 15.6 WU. The patient had previously been referred for lung transplantation but was denied due to cachexia and lack of social support. Despite aggressive medical therapy, the patient continued to report NYHA Class III/IV symptoms, a 6MWD of 290 meters, an elevated NT-pro BNP in the range of 4,926-15,161 pg/mL and 4 hospitalizations for progressive RV failure and palliative paracenteses over the 15 months preceding FK-506 initiation (**Figure 1**). After discussion with the patient and given the lack of further therapeutic options, she was offered FK-506 on a compassionate basis with a goal trough tacrolimus blood level of 1.5-2.5 ng/mL but without further increase in her targeted PAH therapies. The patient did not agree to have a repeat RHC at the time of FK-506 initiation.

Within 1 month of FK-506 initiation, patient #2 reported some improvement in symptoms and exercise capacity (**Figure 1**). There was an associated decline in NT-pro BNP from 2,669 to 1,895 pg/mL. Within 3 months of treatment, the patient's 6MWD improved by 18 meters, her NT-pro BNP decreased further to 1,580 pg/mL and she reported stable NYHA III symptoms. Her cMRI at baseline, 3 and 6 months showed substantial improvement in RVEF, stable RVEDVi and improvement in RVSVi and CI. Her REVEAL risk score decreased modestly from 12 to 11. As with patient #1, while the 15 months prior to FK-506 were characterized by 4 hospitalizations for RV failure, the subsequent 12 months after initiation of FK-506, were free from any PAH related hospitalizations (**Figure 1**). At 12 months follow-up, she had stable NYHA III symptoms, a 94 meter increase in 6MWD, an NT-pro BNP reduced to 1,895 pg/mL (30% reduction compared to baseline), and improved hemodynamics: mRAP 7 mmHg, mPAP 58 mmHg, PAWP 10 mmHg, CO 3.4 L/min, SVO2 59%, and PVR 14.1 WU.

**Patient #3:** A 55-year-old female with severe end-stage drugs and toxins associated PAH, with NYHA III/IV symptoms on high dose IV treprostinil 140 ng/kg/min, sildenafil 40 mg tid, but intolerance to endothelin receptor antagonists (ERAs) was referred and listed for double lung transplantation in 4/2012. Her most recent RHC (4/2010) demonstrated mRAP 13 mmHg, mPAP 60 mmHg, PAWP 10 mmHg, CO 3.6 L/min and PVR 15 WU. Given the lack of further therapeutic options, she was offered FK-506 on a compassionate basis aiming for the trough tacrolimus blood levels described above. There was no further increase in her PAH-specific therapies. Despite initially successful symptomatic improvement in symptoms over a 5-months period (**Figure 1**) the patient voluntarily discontinued FK-506 citing a stressful family situation. Unfortunately the ensuing 7 months were characterized by progressive clinical worsening culminating in an acute admission into the coronary care unit for overt right heart failure (**Figure 1**).

We conclude that overall patients tolerated low-dose tacrolimus very well and did not report an increase of infections, which is especially important as all three patients were treated with continuous intravenous prostanoids. All three patients had an increase in their 6MWD as well as a decrease in their NT-pro BNP in the first 3-6 months. Most strikingly all 3 patients improved their REVEAL risk score, a composite score of different clinical parameters that predicts survival. None of the patients was admitted to the hospital for worsening of heart failure during the treatment with tacrolimus. Thus, the use of an agent that increases the BMPR2 signaling is efficacious for treating PAH.

## Claims

1. A combination for use in a method of treating or preventing pulmonary hypertension comprising,
tacrolimus or a pharmaceutically acceptable solvate or salt thereof, and
a second active agent effective for treatment of the pulmonary hypertension condition or a condition related thereto,
wherein the second active agent is a phosphodiesterase 5 (PDE5) inhibitor, and wherein the patient experiences at least one of (a) adjustment of one or more hemodynamic parameters indicative of improvement of the pulmonary hypertension condition towards a more normal level versus baseline; (b) increase in exercise capacity versus baseline; (c) lowering of Borg dyspnea index (BDI) versus baseline; (d) improvement of one or more quality of life parameters versus baseline; (e) movement to a lower WHO functional class; and (f) a reduction in plasma natriuretic peptide levels versus baseline.

2. The combination for use, according to claim 1, wherein the pulmonary hypertension:
(a) is associated with one or more of chronic obstructive pulmonary disease (COPD), sleep-disordered breathing, an alveolar hypoventilation disorder, chronic exposure to high altitude, a developmental abnormality, thromboembolic obstruction of proximal and/or distal pulmonary arteries, a non-thrombotic pulmonary embolism, sarcoidosis, histiocytosis X, lymphangiomatosis, or compression of pulmonary vessels; or
(b) is pulmonary arterial hypertension (PAH).

3. The combination for use, according to claim 1, wherein the daily tacrolimus dose provides serum concentration of about 0.02 ng/mL to about 10 ng/mL, preferably, about 0.1 ng/mL to about 5 ng/mL, preferably, about 0.1 ng/mL to about 4 ng/mL.

4. The combination for use, according to claim 1, and wherein the second active agent further comprises at least one drug selected from the group consisting of a prostanoid, a guanylate cyclase activator, a calcium channel blocker, a diuretic, an anticoagulant, oxygen, and a combination thereof.

5. The combination for use, according to claim 1, wherein the PDE5 inhibitor is avanafil, lodenafil, mirodenafil, sildenafil, tadalafil, verdenafil, udenafil, zaprinast, or combinations thereof.

6. The combination for use, according to claim 5, wherein the PDE5 inhibitor is avanafil, udenafil, or a pharmaceutically acceptable solvate or salt thereof.

7. The combination for use, according to claim 5, wherein the PDE5 inhibitor is avanafil or a pharmaceutically acceptable solvate or salt thereof.

## Patentansprüche

1. Kombination zur Verwendung in einem Verfahren zur Behandlung oder Verhinderung von pulmonaler Hypertonie, umfassend:
Tacrolimus oder ein pharmazeutisch akzeptables Solvat oder Salz davon, und
einen zweiten Wirkstoff, der zur Behandlung des pulmonalen Hypertoniezustands oder einer damit zusammenhängenden Erkrankung wirksam ist,
wobei der zweite Wirkstoff ein Phosphodiesterase-5-(PDE5-) Inhibitor ist, und
wobei bei dem Patienten mindestens eines der Folgenden eintritt: (a) Einstellung von ein oder mehreren hämodynamischen Parametern, die auf eine Verbesserung des pulmonalen Hypertoniezustands zu einem normaleren Niveau im Vergleich zu Baseline hinweisen; (b) Erhöhung der Belastungsfähigkeit im Vergleich zu Baseline; (c) Senkung des Borg-Dyspnoe-Index (BDI) im Vergleich zu Baseline; (d) Verbesserung von ein oder mehreren Parametern zur Lebensqualität im Vergleich zu Baseline; (e) Verschiebung in eine geringere WHO-Funktionsklasse; und (f) eine Verringerung der natriuretischen Peptidspiegel im Plasma im Vergleich zu Baseline.

2. Kombination zur Verwendung nach Anspruch 1, wobei die pulmonale Hypertonie:
(a) mit einem oder mehreren von chronischobstruktiver Lungenerkrankung (COPD), schlafbezogener Atmungsstörung, einer alveolären Hypoventilationsstörung, chronischer Exposition gegenüber großer Höhe, einer Entwicklungsanomalie, thromboembolischer Obstruktion proximaler und/oder distaler Lungenarterien, einer nicht-thrombotischen Lungenembolie, Sarkoidose, Histiozytose X, Lymphangiomatose oder Kompression von Lungengefäßen assoziiert ist; oder
(b) pulmonal-arterielle Hypertonie (PAH) ist.

3. Kombination zur Verwendung nach Anspruch 1, wobei die Tagesdosis von Tacrolimus eine Serumkonzentration von ungefähr 0,02 ng/ml bis ungefähr 10 ng/ml, vorzugsweise ungefähr 0,1 ng/ml bis ungefähr 5 ng/ml, vorzugsweise ungefähr 0,1 ng/ml bis ungefähr 4 ng/ml bereitstellt.

4. Kombination zur Verwendung nach Anspruch 1, und wobei der zweite Wirkstoff ferner mindestens ein Arzneimittel umfasst, das aus der aus einem Prostanoid, einem Guanylatcyclase-Aktivator, einem Kalziumkanalblocker, einem Diuretikum, einem Antikoagulanz, Sauerstoff und einer Kombination davon bestehenden Gruppe ausgewählt ist.

5. Kombination zur Verwendung nach Anspruch 1, wobei der PDES-Inhibitor Avanafil, Lodenafil, Mirodenafil, Sildenafil, Tadalafil, Verdenafil, Udenafil, Zaprinast oder Kombinationen davon ist.

6. Kombination zur Verwendung nach Anspruch 5, wobei der PDES-Inhibitor Avanafil, Udenafil oder ein pharmazeutisch akzeptables Solvat oder Salz davon ist.

7. Kombination zur Verwendung nach Anspruch 5, wobei der PDES-Inhibitor Avanafil oder ein pharmazeutisch akzeptables Solvat oder Salz davon ist.

## Revendications

1. Combinaison pour utilisation dans un procédé de traitement ou de prévention de l'hypertension pulmonaire comprenant,
le tacrolimus ou un solvate ou sel pharmaceutiquement acceptable de celui-ci, et un deuxième agent actif efficace pour le traitement de l'affection d'hypertension pulmonaire ou une affection liée à celle-ci,
dans laquelle le deuxième agent actif est un inhibiteur de phosphodiestérase 5 (PDE5), et
le patient présentant au moins l'un parmi (a) l'ajustement d'un ou plusieurs paramètres hémodynamiques indicatifs d'une amélioration de l'affection d'hypertension pulmonaire vers un niveau plus normal par rapport aux valeurs de référence ; (b) une augmentation de la capacité d'effort par rapport aux valeurs de référence ; (c) une diminution de l'indice de dyspnée de Borg (BDI) par rapport aux valeurs de référence ;
(d) une amélioration d'un ou plusieurs paramètres de qualité de vie par rapport aux valeurs de référence ; (e) un déplacement vers une classe fonctionnelle de l'OMS inférieure ; et (f) une réduction des taux plasmatiques de peptide natriurétique par rapport aux valeurs de référence.

2. Combinaison pour utilisation, selon la revendication 1, l'hypertension pulmonaire étant :
(a) associée à l'un ou plusieurs parmi la bronchopneumopathie chronique obstructive (BPCO), des troubles respiratoires du sommeil, un trouble d'hypoventilation alvéolaire, une exposition chronique à la haute altitude, une anomalie du développement, une obstruction thrombo-embolique des artères pulmonaires proximales et/ou distales, une embolie pulmonaire non thrombotique, la sarcoïdose, l'histiocytose X, la lymphangiomatose ou une compression des vaisseaux pulmonaires ; ou
(b) est une hypertension artérielle pulmonaire(HAP).

3. Combinaison pour utilisation, selon la revendication 1, la dose journalière de tacrolimus produisant une concentration sérique d'environ 0,02 ng/ml à environ 10 ng/ml, de préférence, environ 0,1 ng/ml à environ 5 ng/ml, de préférence, environ 0,1 ng/ml à environ 4 ng/ml.

4. Combinaison pour utilisation, selon la revendication 1, et dans laquelle le deuxième agent actif comprend en outre au moins un médicament choisi dans le groupe constitué d'un prostanoïde, un activateur de la guanylate cyclase, un inhibiteur calcique, un diurétique, un anticoagulant, l'oxygène, et une combinaison de ceux-ci .

5. Combinaison pour utilisation, selon la revendication 1, dans laquelle l'inhibiteur de PDE5 est l'avanafil, le lodénafil, le mirodénafil, le sildénafil, le tadalafil, le verdénafil, l'udénafil, le zaprinast, ou des combinaisons de ceux-ci.

6. Combinaison pour utilisation, selon la revendication 5, l'inhibiteur de PDE5 étant l'avanafil, l'udénafil, ou un solvate ou sel pharmaceutiquement acceptable de ceux-ci.

7. Combinaison pour utilisation, selon la revendication 5, dans laquelle l'inhibiteur de PDE5 est l'avanafil ou un solvate ou sel pharmaceutiquement acceptable de celui-ci.
